# EUROPEAN PATENT APPLICATION

(11) **EP 4 209 577 A1**
(43) Date of publication of application: **12.07.2023**
(21) Application number: 21864140.5
(22) Date of filing: 20.08.2021
(51) Int. Cl.: C12N 1/20, A23L 33/135, C12N 15/11

(54) **AGENT FOR INCREASING CONTENT OF IGA ANTIBODY IN MILK**

(30) Priority: 01.09.2020 JP 2020146884
(71) Applicant: Tohoku University, Sendai-shi, Miyagi 980-8577 (JP); The University of Tokyo, Bunkyo-ku, Tokyo 113-8654 (JP)
(72) Inventor: NOCHI, Tomonori, Sendai-shi, Miyagi 980-8577 (JP); USAMI, Katsuki, Sendai-shi, Miyagi 980-8577 (JP); ASO, Hisashi, Sendai-shi, Miyagi 980-8577 (JP); KIYONO, Hiroshi, Tokyo 113-8654 (JP); FUJIHASHI, Kohtaro, Tokyo 113-8654 (JP); SATO, Shintaro, Tokyo 113-8654 (JP)
(74) Representative: Hamer, Christopher K.
(86) International application number: PCT/JP2021/030571
(87) International publication number: WO 2022/050082

(57) **Abstract**

It is to provide intestinal symbiotic bacteria having an action of increasing an IgA content in milk. An agent containing one or more bacterial strains selected from: a bacterial strain of the genus *Bacteroides* which has a 16S rRNA gene having at least 90% identity to the nucleotide sequence represented by SEQ ID NO: 1, and has an action of increasing an IgA antibody content in milk; and a bacterial strain of the genus *Prevotella* which has a 16S rRNA gene having at least 90% identity to the nucleotide sequence represented by SEQ ID NO: 2, and has an action of increasing an IgA antibody content in milk is used as an agent for increasing an IgA antibody content in milk.

## Description

### Technical Field

The present invention relates to an agent for increasing an IgA antibody (hereinafter, also referred to as "IgA") content in milk, containing intestinal symbiotic bacteria having an action of increasing an IgA content in milk as an active ingredient.

### Background Art

Microbes are originally foreign matter to host organisms and therefore may become targets to be eliminated by the immune system. However, intestinal symbiotic bacteria are characterized by living symbiotically in the gut without undergoing aggressive elimination by the gut immune system. Mammals including humans are infected with various bacteria through contact with the outside world after birth in an aseptic state so that intestinal bacterial flora is formed. Approximately 1000 or more species including approximately 100 trillion or more intestinal symbiotic bacteria are considered to exist in the human gut and largely influence an environment in the gut.

Bacteria of the genus *Bacteroides* have heretofore been considered as low useful opportunistic bacteria. Also, these bacteria are anaerobic bacteria and are therefore difficult to culture, and much about the bacteria, including proliferation mechanisms, has remained unknown. From recent research on the bacteria of the genus *Bacteroides,* it has been reported that when Peyer's patch cells of gut lymphoid tissues are cultured in the presence of heat-killed bacteria of the genus *Bacteroides,* the amount of IgA secreted into a culture supernatant is larger as compared with when cultured in the presence of heat-killed bacteria of the genus *Lactobacillus* which are lactic acid bacteria (non-patent documents 1 and 2). It has also been reported that *Bacteroides acidifaciens* has a therapeutic effect on metabolic disease caused by abnormal glucose and lipid metabolism (patent document 1). However, the relation of intestinal symbiotic bacteria (i.e., bacteria constituting intestinal bacterial flora) to IgA antibody contents in milk has not been known so far.

### Prior Art Documents

### Patent Document

Patent document 1: Japanese unexamined Patent Application Publication (Translation of PCT Application) No. 2017-538702

### Non-patent Documents

Non-patent document 1: Journal of Intestinal Microbiology 27: 203-209, 2013
Non-patent document 2: Biosci Biotechnol Biochem. 2009 Feb; 73 (2): 372-7

### Summary of the Invention

### Object to be Solved by the Invention

An object of the present invention is to provide intestinal symbiotic bacteria having action of increasing an IgA content in milk.

### Means to Solve the Object

The present inventors have continued diligent studies to attain the object. During the process, the present inventors have first found that in a breast-feeding mammalian mother, an immune function in Peyer's patches (hereinafter, also referred to as "PP") is activated so that IgA-producing plasma cells migrate into the mammary gland from PP, resulting in an increased IgA content in milk. The present inventors have also confirmed that decrease in the ratio of a bacterial strain of the genus *Bacteroides* (*Bacteroides acidifaciens*) and a bacterial strain of the genus *Prevotella* (*Prevotella buccalis*) to the whole intestinal bacterial flora of a breast-feeding mammalian mother decreases the number of IgA-producing plasma cells in the mammary gland and an IgA content in milk. The present inventors have further found that the oral inoculation of established cell lines of these bacterial species to a breast-feeding mammalian mother with a decreased ratio of these bacterial species to the whole intestinal bacterial flora increases the number of IgA-producing plasma cells present in the mammary gland and an IgA content in milk. The present invention has been completed on the basis of these findings.

Specifically, the present invention is as follows.
[1] An agent for increasing an IgA antibody content in milk, comprising one or more bacterial strains selected from:
   a bacterial strain of the genus *Bacteroides* which has a 16S rRNA gene having at least 90% identity to the nucleotide sequence represented by SEQ ID NO: 1, and has an action of increasing an IgA antibody content in milk; and
   a bacterial strain of the genus *Prevotella* which has a 16S rRNA gene having at least 90% identity to the nucleotide sequence represented by SEQ ID NO: 2, and has an action of increasing an IgA antibody content in milk.
[2] The agent according to the above [1], wherein the bacterial strain has an action of activating an immune function in Peyer's patches, and increasing the number of IgA antibody-producing plasma cells.
[3] The agent according to the above [1] or [2], wherein the increasing agent is inoculated to a mammalian mother in which a ratio of the bacterial strain of the genus *Bacteroides* having a 16S rRNA gene having at least 90% identity to the nucleotide sequence represented by SEQ ID NO: 1, and the bacterial strain of the genus *Prevotella* having a 16S rRNA gene having at least 90% identity to the nucleotide sequence represented by SEQ ID NO: 2 in intestinal bacterial flora is decreased.
[4] The agent according to any one of the above [1] to [3], wherein the agent is orally inoculated.

Other exemplary embodiments of the present invention can be:
a method for increasing an IgA antibody content in milk, comprising the step of inoculating one or more bacterial strains (hereinafter, these are also collectively referred to as the "present bacterial strain") selected from: a bacterial strain of the genus *Bacteroides* which has a 16S rRNA gene having at least 90% identity to the nucleotide sequence represented by SEQ ID NO: 1, and has an action of increasing an IgA antibody content in milk (hereinafter, also referred to as the "present bacterial strain of the genus *Bacteroides*"); and a bacterial strain of the genus *Prevotella* which has a 16S rRNA gene having at least 90% identity to the nucleotide sequence represented by SEQ ID NO: 2, and has an action of increasing an IgA antibody content in milk (hereinafter, also referred to as the "present bacterial strain of the genus *Prevotella*") to a mammalian mother in need of increase in IgA antibody content in milk;
one or more present bacterial strains for use in increase in IgA antibody content in milk; and use of one or more present bacterial strains for producing an agent for increasing an IgA antibody content in milk.

### Effect of the Invention

The present bacterial strain has an action of increasing an IgA content in milk. Particularly, when the present bacterial strain is inoculated to a breast-feeding mammalian mother with a decreased IgA content in milk as a result of decrease in the ratio of the present bacterial strain to the whole intestinal bacterial flora due to a factor that disturbs intestinal bacterial flora, such as stress or the administration of an antibiotic, the IgA content in milk is increased owing to the effect of the present bacterial strain. Therefore, the pathogenic bacterial infection or viral infection of a child in infancy (specifically, a period for which the ability to self-produce IgA is absent or low) can be effectively prevented by ingesting the milk. This contributes to improvement in productivity in the livestock industry.

### Brief Description of Drawings

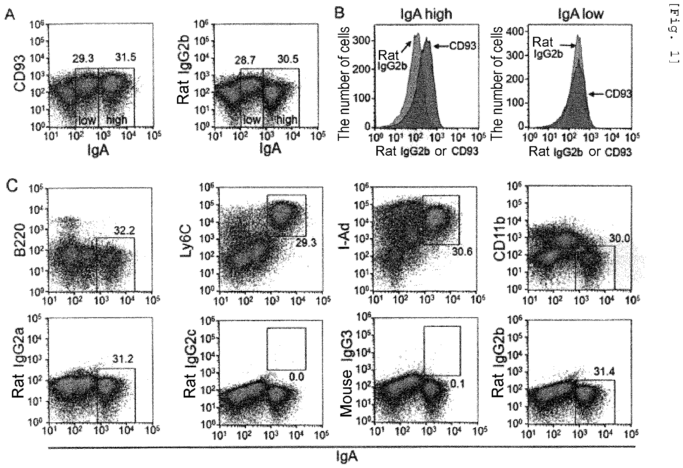

[Figure 1] Each of Figures 1A and 1B is a diagram showing results of analyzing an IgA antibody production level and an expression level of a plasma cell marker CD93 as to cells in the mammary gland. The numeric values in Figure 1A depict the ratios (%) of cells included in the boxed areas to the whole cells. "IgA high" of Figure 1B depicts cells included in the "high" area in the boxed areas of Figure 1A (i.e., plasma cells producing a high level of an IgA antibody). "IgA low" of Figure 1B depicts cells included in the "low" area in the boxed areas of Figure 1A (i.e., cells producing a low level of an IgA antibody). Figure 1C is a diagram showing results of analyzing an IgA antibody production level and expression levels of 4 types of cell surface markers (B220, Ly6C, I-Ad, and CD11b) as to cells in the mammary gland. The boxed areas in Figure 1C depict plasma cells producing a high level of an IgA antibody. The numeric values in Figure 1C depict the ratios (%) of plasma cells producing a high level of an IgA antibody to the whole cells.

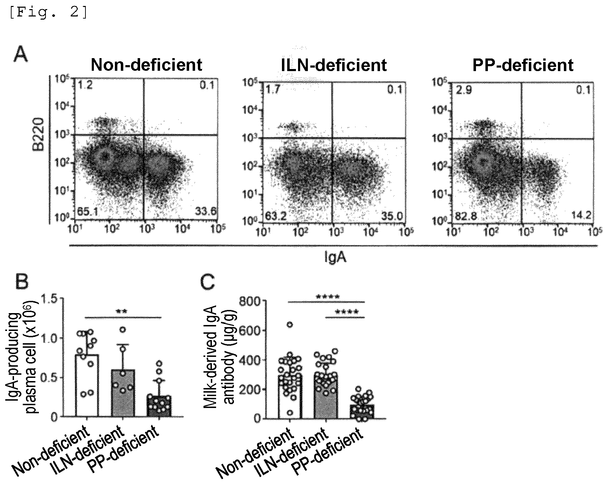

[Figure 2] Figure 2A is a diagram showing results of analyzing the abundance ratio of plasma cells in the mammary gland of 3 types of inbred (BALB/c) mother mice (a BALB/c mouse ["non-deficient" in the diagram], an inguinal lymph node (hereinafter, also referred to as "ILN")-deficient mouse ["ILN-deficient" in the diagram], and a PP-deficient mouse ["PP-deficient" in the diagram]) with IgA and B220 as markers. Figure 2B is a diagram showing results (mean + standard deviation) of measuring the number of IgA-producing plasma cells (cells included in the lower right area of each graph in Figure 2A) present in the mammary gland on the basis of the results of Figure 2A. Figure 2C is a diagram showing results (mean + standard deviation) of measuring an IgA antibody concentration in the gastric contents of child mice that ingested the milk of the 3 types of inbred (BALB/c) mother mice. "**" and "****" in the diagram each depict statistically significant difference (p < 0.01 and p < 0.0001) (the same holds true for the description below). The open circles (o) in the diagram depict respective samples (the same holds true for the description below).

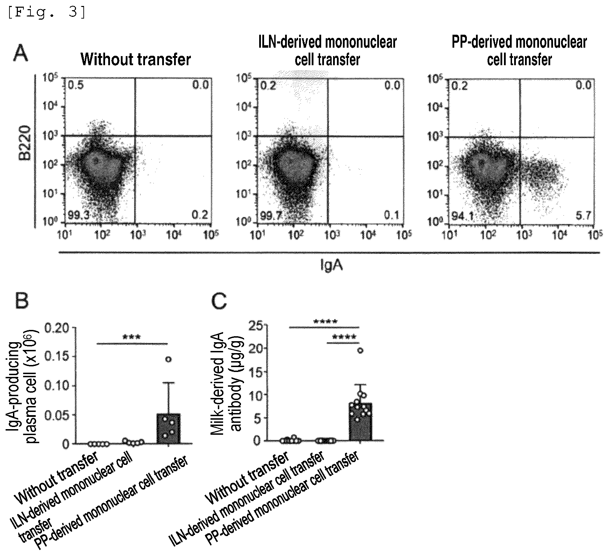

[Figure 3] Figure 3A is a diagram showing results of analyzing plasma cells in the mammary gland of 3 types of immunodeficient (C.B-17/Icr-scid/scidJcl) mother mice (an immunodeficient mouse to which ILN-derived mononuclear cells of a wild-type C.B-17/Icr-+/+Jcl mouse [hereinafter, also referred to as a "wild-type (Icr^{+/+}) mouse"] were transferred ["ILN-derived mononuclear cell transfer" in the diagram]; an immunodeficient mouse to which PP-derived mononuclear cells of a wild-type mouse were transferred ["PP-derived mononuclear cell transfer" in the diagram]; and an immunodeficient mouse without transfer of these cells ["without transfer" in the diagram]) with IgA and B220 as markers. Figure 3B is a diagram showing results (mean + standard deviation) of measuring the number of IgA-producing plasma cells (cells included in the lower right area of each graph in Figure 3A) on the basis of the results of Figure 3A. Figure 3C is a diagram showing results (mean + standard deviation) of measuring an IgA antibody concentration in the gastric contents of child mice that ingested the milk of the 3 types of immunodeficient (C.B-17/Icr-scid/scidJcl) mother mice. "***" in the diagram depicts statistically significant difference (p < 0.001) (the same holds true for the description below). In the present Examples, statistical processing was performed by a multiple comparison test using Prism 7 software.

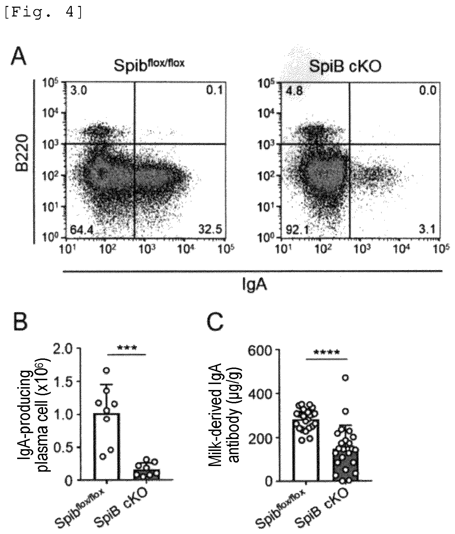

[Figure 4] Figure 4A is a diagram showing results of analyzing plasma cells in the mammary gland of 2 types of inbred (C57BL/6) mother mice (a Spib^{flox/flox} mouse ["Spib^{flox/flox}" in the diagram]; and a gut epithelium-specifically SpiB gene-deficient mouse [hereinafter, also referred to as a "SpiB cKO mouse"] prepared on the basis of the Spib^{flox/flox} mouse ["SpiB cKO" in the diagram]) with IgA and B220 as markers. Figure 4B is a diagram showing results (mean + standard deviation) of measuring the number of IgA-producing plasma cells (cells included in the lower right area of each graph in Figure 4A) on the basis of the results of Figure 4A. Figure 4C is a diagram showing results (mean + standard deviation) of measuring an IgA antibody concentration in the gastric contents of child mice that ingested the milk of the 2 types of inbred (C57BL/6) mother mice.

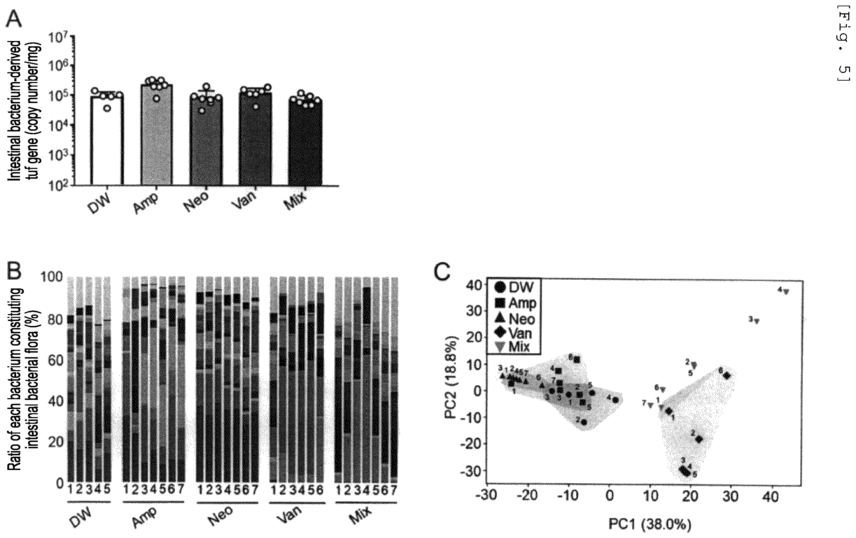

[Figure 5] Figure 5 is a diagram showing results of analyzing the total number of intestinal bacteria with bacterium-derived tuf gene as an index (Figure 5A), and the ratio of each bacterium constituting intestinal bacterial flora (Figures 5B and 5C) after inbred (BALB/c) mother mice were allowed to freely drink water containing any of 3 types of antibiotics (ampicillin ["Amp" in the diagram, neomycin ["Neo" in the diagram], and vancomycin ["Van" in the diagram]]) or a mixture thereof ("Mix" in the diagram), or antibiotic-free water ("DW" in the diagram). In Figure 5A, the ordinate shows the copy number of the tuf gene derived from each bacterium per mg of stool. The numeric values (1 to 7) in Figures 5B and 5C depict each individual mouse.

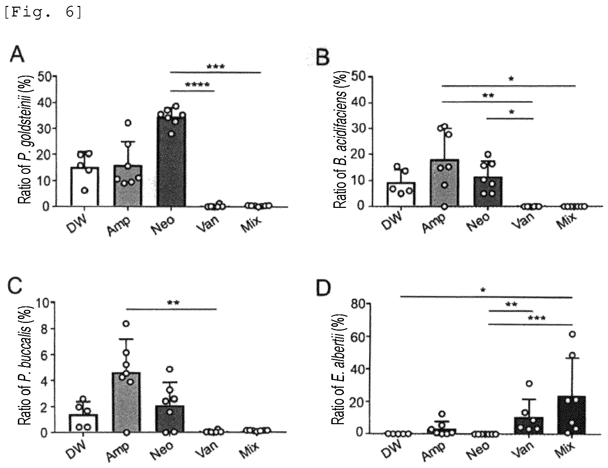

[Figure 6] Figure 6 is a diagram showing results (mean + standard deviation) of measuring the ratios of 4 types of bacterial strains with a particularly largely changed ratio *(Parabacteroides goldsteinii* [Figure 6A], *Bacteroides acidifaciens* [Figure 6B], *Prevotella buccalis* [Figure 6C], and *Escherichia albertii* [Figure 6D]) to intestinal bacterial flora after inbred (BALB/c) mother mice were allowed to freely drink water containing any of 3 types of antibiotics (ampicillin ["Amp" in the diagram, neomycin ["Neo" in the diagram], and vancomycin ["Van" in the diagram]]) or a mixture thereof ("Mix" in the diagram), or antibiotic-free water ("DW" in the diagram). "*" in the diagram depicts statistically significant difference (p < 0.05) (the same holds true for the description below).

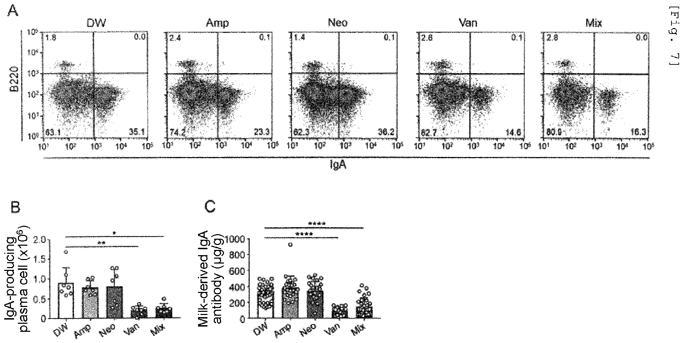

[Figure 7] Figure 7A is a diagram showing results of analyzing plasma cells in the mammary gland with IgA and B220 as markers after inbred (BALB/c) mother mice were allowed to freely drink 5 types of waters (water containing ampicillin ["Amp" in the diagram; water containing neomycin ["Neo" in the diagram]; water containing vancomycin ["Van" in the diagram]); water containing a mixture of these 3 types of antibiotics ("Mix" in the diagram); and antibiotic-free water ("DW" in the diagram). Figure 7B is a diagram showing results (mean + standard deviation) of measuring the number of IgA-producing plasma cells (cells included in the lower right area of each graph in Figure 7A) on the basis of the results of Figure 7A. Figure 7C is a diagram showing results (mean + standard deviation) of measuring an IgA antibody concentration in the gastric contents of child mice that ingested the milk of the inbred (BALB/c) mother mice that freely drank each water.

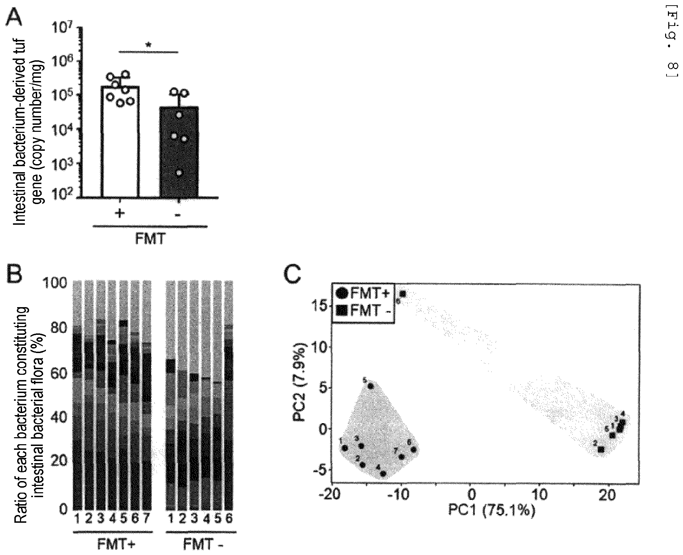

[Figure 8] Figure 8 is a diagram showing results of analyzing the total number of intestinal bacteria with bacterium-derived tuf gene as an index (Figure 8A), and the ratio of each bacterium constituting intestinal bacterial flora (Figures 8B and 8C) by performing fecal microbiota transplantation (FMT) using the stool of a breast-feeding healthy inbred (BALB/c) mother mouse ("FMT +" in the diagram) or without performing FMT ("FMT -" in the diagram) after inbred (BALB/c) mother mice were allowed to freely drink water containing a mixture of 3 types of antibiotics (ampicillin, neomycin, and vancomycin) (hereinafter, also simply referred to as an "antibiotic mixture"). In Figure 8A, the ordinate shows the copy number of the tuf gene derived from each bacterium per mg of stool. The numeric values (1 to 7) in Figures 8B and 8C depict each individual mouse.

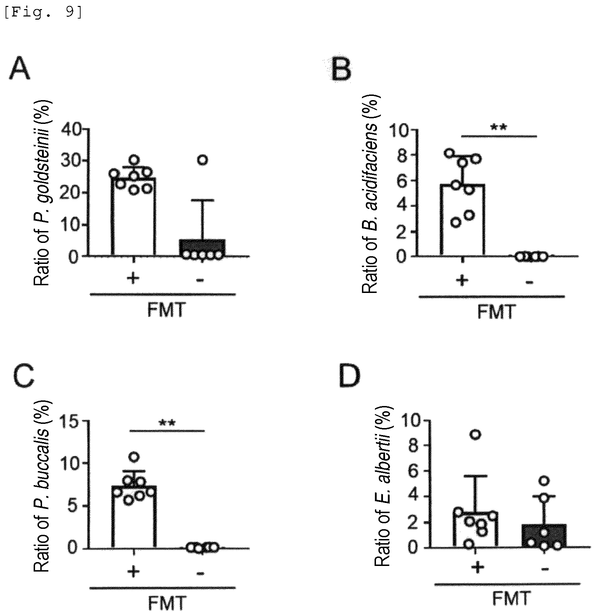

[Figure 9] Figure 9 is a diagram showing results (mean + standard deviation) of measuring the ratios of 4 types of bacterial strains *(Parabacteroides goldsteinii* [Figure 9A], *Bacteroides acidifaciens* [Figure 9B], *Prevotella buccalis* [Figure 9C], and *Escherichia albertii* [Figure 9D]) to intestinal bacterial flora by performing FMT using the stool of a breast-feeding healthy inbred (BALB/c) mother mouse ("FMT +" in the diagram) or without performing FMT ("FMT - " in the diagram) after inbred (BALB/c) mother mice were allowed to freely drink water containing a mixture of 3 types of antibiotics (ampicillin, neomycin, and vancomycin) (hereinafter, also simply referred to as an "antibiotic mixture").

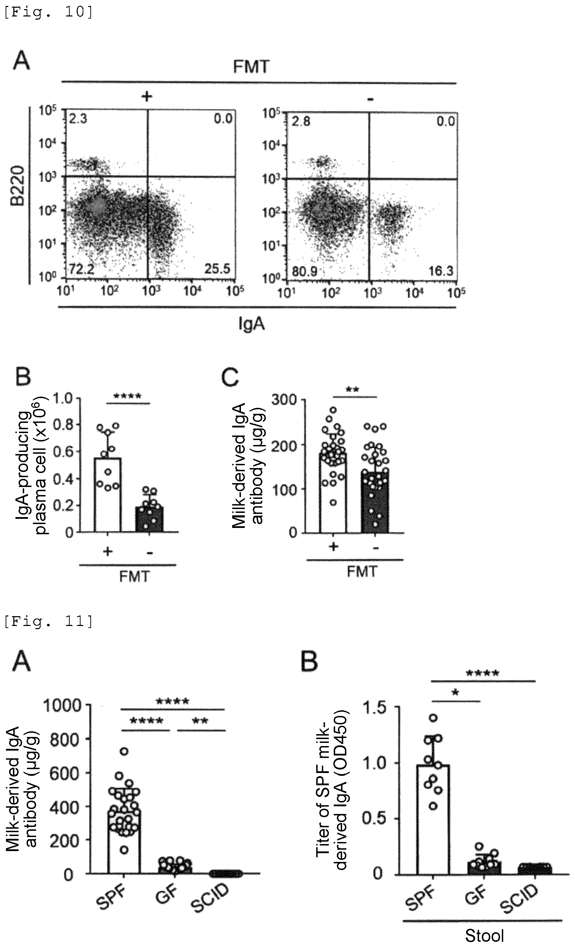

[Figure 10] Figure 10A is a diagram showing results of analyzing plasma cells in the mammary gland with IgA and B220 as markers by performing FMT using the stool of a breast-feeding healthy inbred (BALB/c) mother mouse ("FMT +" in the diagram) or without performing FMT ("FMT -" in the diagram) after inbred (BALB/c) mother mice were allowed to freely drink water containing a mixture of the 3 types of antibiotics. Figure 10B is a diagram showing results (mean + standard deviation) of measuring the number of IgA-producing plasma cells (cells included in the lower right area of each graph in Figure 10A) on the basis of the results of Figure 10A. Figure 10C is a diagram showing results (mean + standard deviation) of measuring an IgA antibody concentration in the gastric contents of child mice that ingested the milk of the inbred (BALB/c) mother mice that underwent FMT ("FMT +" in the diagram) or did not undergo FMT ("FMT -" in the diagram).
[Figure 11] Figure 11A is a diagram showing results (mean + standard deviation) of measuring an IgA antibody concentration in the gastric contents of child mice that ingested the milk of 3 types of mother mice (a SPF mouse ["SPF" in the diagram], a GF mouse ["GF" in the diagram], and an immunodeficient mouse ["SCID" in the diagram]).
Figure 11B is a diagram showing results (mean + standard deviation) of analyzing reactivity with microbes contained in the stool of 3 types of mother mice (a SPF mouse ["SPF" in the diagram], a GF mouse ["GF" in the diagram], and an immunodeficient mouse ["SCID" in the diagram]) as to an IgA antibody in the gastric contents of child mice that ingested the milk of the SPF mouse.

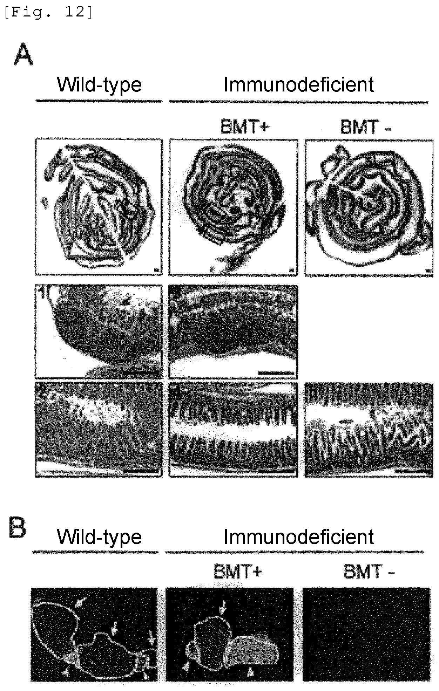

[Figure 12] Figure 12A is a diagram showing results of analyzing the small intestinal tissues of 3 types of mother mice (a wild-type mouse ["wild-type" in the diagram]; an immunodeficient mouse in which bone marrow-derived mononuclear cells of a wild-type mouse were transplanted [BMT; bone marrow transplantation] ["immunodeficient BMT+" in the diagram]; and an immunodeficient mouse without BMT ["immunodeficient BMT-" in the diagram]) by hematoxylin-eosin staining. Each of the middle and lower images (1 to 5) is an enlarged view of the boxed areas (1 to 5) in the upper images. The scale bars in the diagram depicts 500 pm.
Figure 12B is a diagram showing results of analyzing the small intestinal tissues of the 3 types of mother mice by an immunohistological staining method using an anti-CD3 antibody for detecting T cells and an anti-B220 antibody for detecting B cells. The areas surrounded by white lines, which are indicated by arrows, in the diagram depict the presence of B cells (B220-positive cells), and the areas surrounded by white lines, which are indicated by arrowheads, depict the presence of T cells (CD3-positive cells).

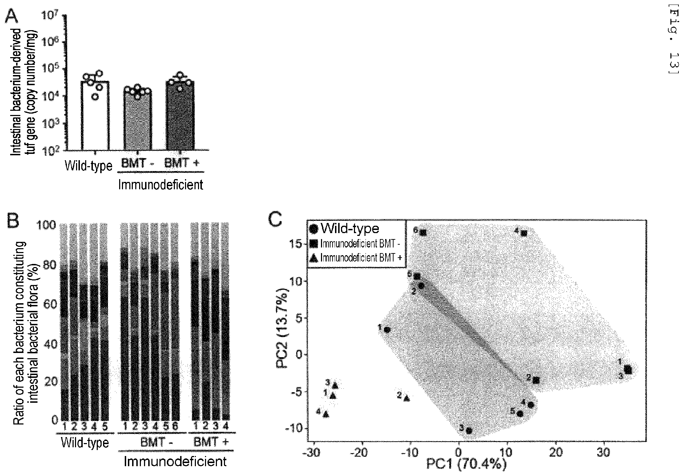

[Figure 13] Figure 13 is a diagram showing results of analyzing the total number of intestinal bacteria with bacterium-derived tuf gene as an index (Figure 13A), and the ratio of each bacterium constituting intestinal bacterial flora (Figures 13B and 13C) as to 3 types of mother mice (a wild-type mouse ["wild-type" in the diagram]; an immunodeficient mouse in which BMT with bone marrow-derived mononuclear cells of a wild-type mouse was performed ["immunodeficient BMT+" in the diagram]; and an immunodeficient mouse without BMT ["immunodeficient BMT-" in the diagram]). In Figure 13A, the ordinate shows the copy number of the tuf gene derived from each bacterium per mg of stool. The numeric values (1 to 6) in Figures 13B and 13C depict each individual mouse.

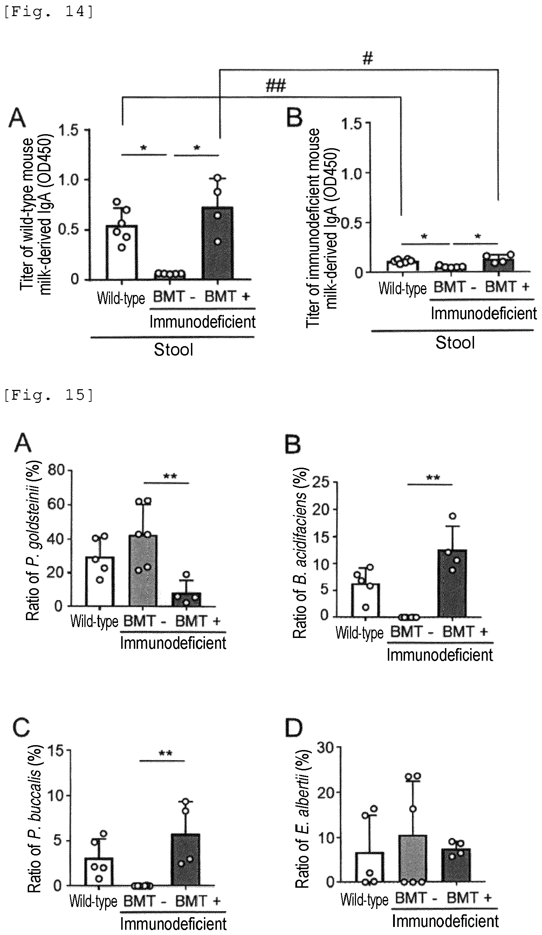

[Figure 14] Figure 14 is a diagram showing results (mean + standard deviation) of analyzing reactivity with microbes contained in the stool of 3 types of mother mice (a wild-type mouse ["wild-type" in the diagram]; an immunodeficient mouse in which BMT with bone marrow mononuclear cells derived from a wild-type mouse was performed ["immunodeficient BMT+" in the diagram]; and an immunodeficient mouse without BMT ["immunodeficient BMT-" in the diagram]) as to an IgA antibody in the gastric contents of child mice that ingested the milk of 2 types of mother mice (a wild-type mouse [Figure 14A] and an immunodeficient mouse [Figure 14B]). "#" and "##" in the diagram depict statistically significant difference (p < 0.05 and p < 0.01, respectively) by t-test.
[Figure 15] Figure 15 is a diagram showing results (mean + standard deviation) of measuring the ratios of 4 types of bacterial strains *(Parabacteroides goldsteinii* [Figure 15A], *Bacteroides acidifaciens* [Figure 15B], *Prevotella buccalis* [Figure 15C], and *Escherichia albertii* [Figure 15D]) to intestinal bacterial flora as to 3 types of mother mice (a wild-type mouse ["wild-type" in the diagram]; an immunodeficient mouse in which BMT with bone marrow mononuclear cells derived from a wild-type mouse was performed ["immunodeficient BMT+" in the diagram]; and an immunodeficient mouse without BMT ["immunodeficient BMT-" in the diagram]).

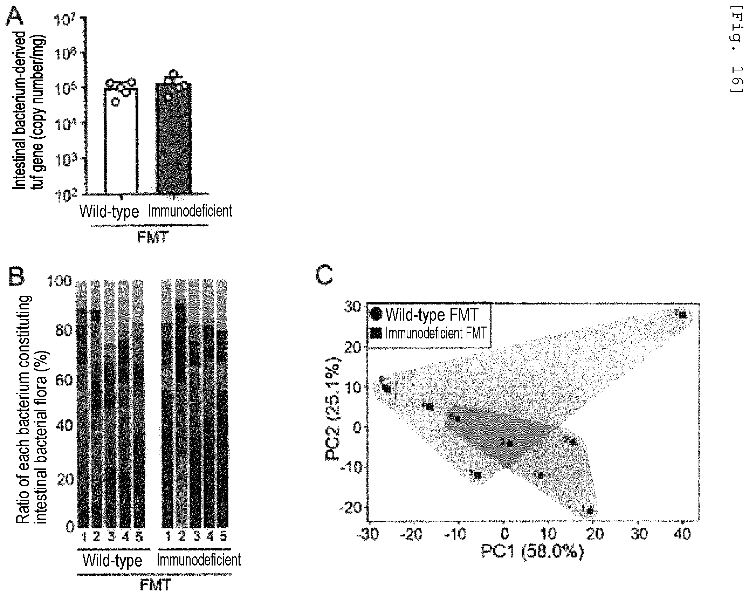

[Figure 16] Figure 16 is a diagram showing results of analyzing the total number of intestinal bacteria with bacterium-derived tuf gene as an index (Figure 16A), and the ratio of each bacterium constituting intestinal bacterial flora (Figures 16B and 16C) by performing FMT using stool derived from 2 types of mice (a wild-type C.B-17/Icr-+/+Jcl mouse and an immunodeficient [C.B-17/Icr-scid/scidJcl] mouse) ("wild-type FMT" and "immunodeficient FMT" in the diagram) after inbred (BALB/c) mother mice were allowed to freely drink water containing a mixture of 3 types of antibiotics (ampicillin, neomycin, and vancomycin). In Figure 16A, the ordinate shows the copy number of the tuf gene derived from each bacterium per mg of stool. The numeric values (1 to 5) in Figures 16B and 16C depict each individual mouse.

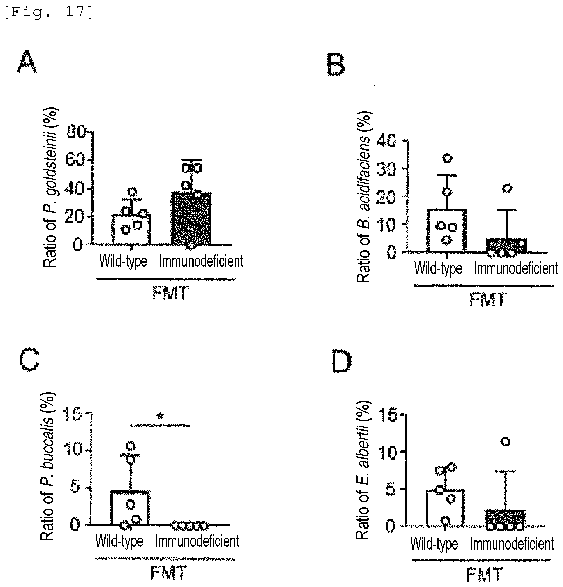

[Figure 17] Figure 17 is a diagram showing results (mean + standard deviation) of measuring the ratios of 4 types of bacterial strains *(Parabacteroides goldsteinii* [Figure 17A], *Bacteroides acidifaciens* [Figure 17B], *Prevotella buccalis* [Figure 17C], and *Escherichia albertii* [Figure 17D]) to intestinal bacterial flora by performing FMT using stool derived from 2 types of mice (a wild-type mouse and an immunodeficient mouse) ("wild-type FMT" and "immunodeficient FMT" in the diagram) after inbred (BALB/c) mother mice were allowed to freely drink water containing a mixture of the 3 types of antibiotics.

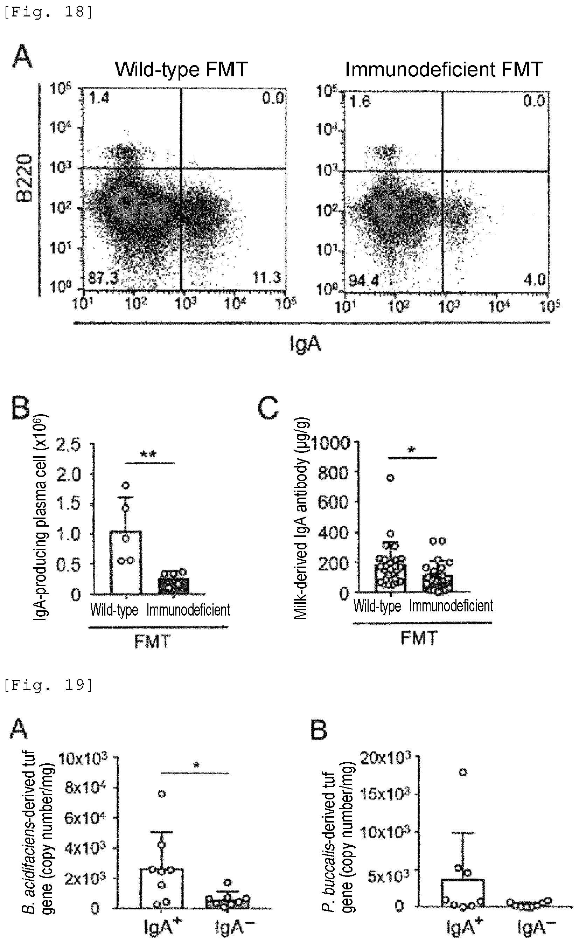

[Figure 18] Figure 18A is a diagram showing results of analyzing an IgA antibody production level and a B220 expression level of plasma cells in the mammary gland by performing FMT using stool derived from 2 types of mice (a wild-type mouse and an immunodeficient mouse) ("wild-type FMT" and "immunodeficient FMT" in the diagram) after inbred (BALB/c) mother mice were allowed to freely drink water containing a mixture of the 3 types of antibiotics. Figure 18B is a diagram showing results (mean + standard deviation) of measuring the number of IgA-producing plasma cells (cells included in the lower right area of each graph in Figure 18A) on the basis of the results of Figure 18A. Figure 18C is a diagram showing results (mean + standard deviation) of measuring an IgA antibody concentration in the gastric contents of child mice that ingested the milk of the 2 types of inbred (BALB/c) mother mice that underwent FMT.
[Figure 19] Figure 19 is a diagram showing results of analyzing the ratios of a bacterial strain that bound to a BALB/c mother mouse milk-derived IgA antibody ("IgA⁺" in the diagram) and a bacterial strain that did not bind thereto ("IgA⁻" in the diagram) with bacterium-derived tuf gene as an index as to 2 types of bacterial strains (*Bacteroides acidifaciens* [Figure 19A] and *Prevotella buccalis* [Figure 19B]). The ordinate shows the copy number of the tuf gene derived from each bacterium per mg of stool.

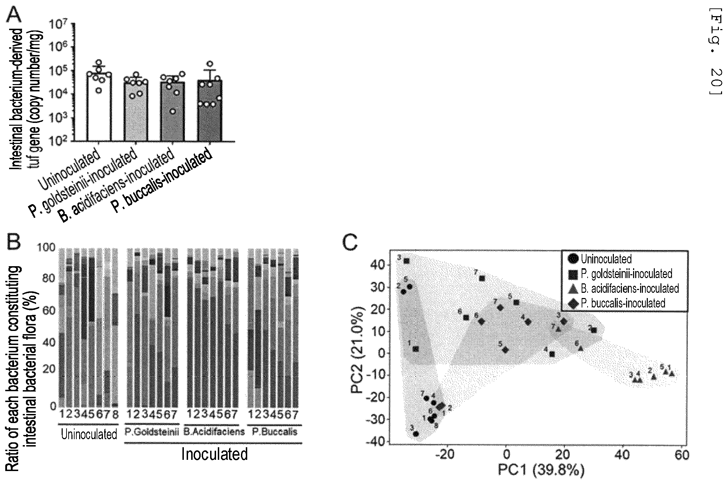

[Figure 20] Figure 20 is a diagram showing results of analyzing the total number of intestinal bacteria with bacterium-derived tuf gene as an index (Figure 20A), and the ratio of each bacterium constituting intestinal bacterial flora (Figures 20B and 20C) after inbred (BALB/c) mother mice were allowed to freely drink water containing an antibiotic mixture and inoculated with 3 types of bacterial strains (*Parabacteroides goldsteinii, Bacteroides acidifaciens,* and *Prevotella buccalis*) ("*P. Goldsteinii-*inoculated", "*B. Acidifaciens*-inoculated."*,* and "*P*. Buccalis-inoculated" in the diagram) or were not inoculated with these bacterial strains ("uninoculated" in the diagram). In Figure 20A, the ordinate shows the copy number of the tuf gene derived from each bacterium per mg of stool. The numeric values (1 to 8) in Figures 20B and 20C depict each individual mouse.

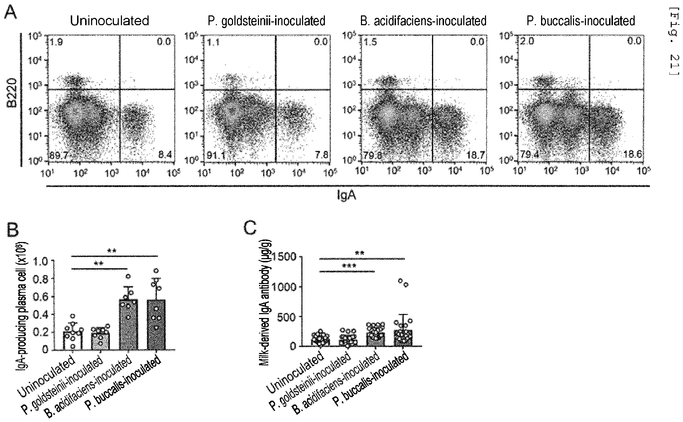

[Figure 21] Figure 21A is a diagram showing results of analyzing plasma cells in the mammary gland with IgA and B220 as markers after inbred (BALB/c) mother mice were allowed to freely drink water containing an antibiotic mixture and inoculated with 3 types of bacterial strains *(Parabacteroides goldsteinii, Bacteroides acidifaciens,* and *Prevotella buccalis*) ("*P. Goldsteinii-*inoculated", "*B.* Acidifaciens-inoculated", and "P. Buccalis-inoculated" in the diagram) or were not inoculated with these bacterial strains ("uninoculated" in the diagram). Figure 21B is a diagram showing results (mean + standard deviation) of measuring the number of IgA-producing plasma cells (cells included in the lower right area of each graph in Figure 21A) on the basis of the results of Figure 21A. Figure 21C is a diagram showing results (mean + standard deviation) of measuring an IgA antibody concentration in the gastric contents of child mice that ingested the milk of the inbred (BALB/c) mother mice inoculated or uninoculated with the 3 types of bacterial strains.

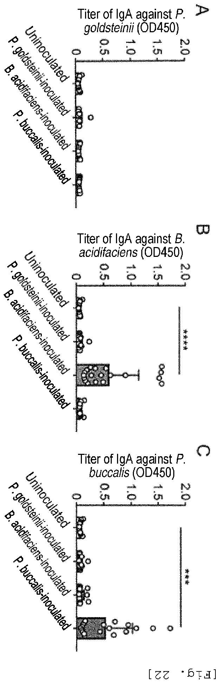

[Figure 22] Figure 22 is a diagram showing results of measuring the titer of an IgA antibody against *Parabacteroides goldsteinii* (Figure 22A), the titer of an IgA antibody *against Bacteroides acidifaciens* (Figure 22B), and the titer of an IgA antibody against *Prevotella buccalis* (Figure 22C) after BALB/c mother mice were allowed to freely drink water containing an antibiotic mixture and inoculated with 3 types of bacterial strains (*Parabacteroides goldsteinii, Bacteroides acidifaciens,* and *Prevotella buccalis) ("P. Goldsteinii-inoculated", "B. Acidifaciens-*inoculated", and "P. *Buccalis-inoculated"* in the diagram) or were not inoculated with these bacterial strains ("uninoculated" in the diagram).

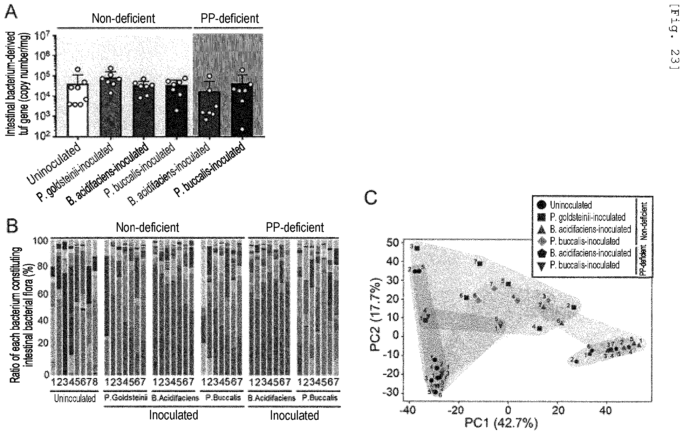

[Figure 23] Figure 23 is a diagram showing results of analyzing the total number of intestinal bacteria with bacterium-derived tuf gene as an index (Figure 23A), and the ratio of each bacterium constituting intestinal bacterial flora (Figures 23B and 23C) after 2 types of inbred (BALB/c) mother mice (a BALB/c mouse ["non-deficient" in the diagram] and a PP-deficient mouse ["PP-deficient" in the diagram]) were allowed to freely drink water containing an antibiotic mixture and inoculated with 3 types of bacterial strains (*Parabacteroides goldsteinii, Bacteroides acidifaciens,* and *Prevotella buccalis) ("P. Goldsteinii-*inoculated", "*B. Acidifaciens-inoculated",* and "*P*. Buccalis-inoculated" in the diagram) or were not inoculated with these bacterial strains ("uninoculated" in the diagram). In Figure 23A, the ordinate shows the copy number of the tuf gene derived from each bacterium per mg of stool. The numeric values (1 to 8) in Figures 23B and 23C depict each individual mouse.

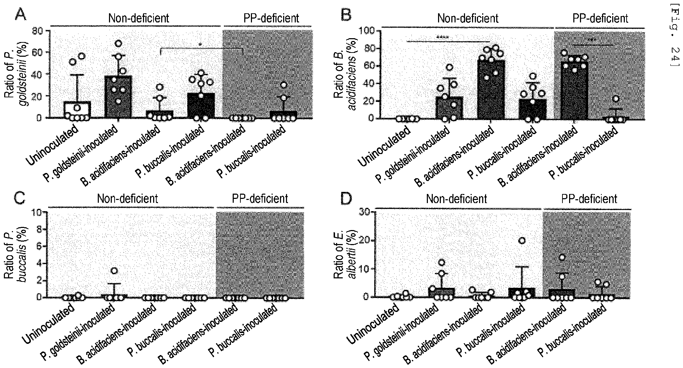

[Figure 24] Figure 24 is a diagram showing results (mean + standard deviation) of measuring the ratios of 4 types of bacterial strains *(Parabacteroides goldsteinii* [Figure 24A], *Bacteroides acidifaciens* [Figure 24B], *Prevotella buccalis* [Figure 24C], and *Escherichia albertii* [Figure 24D]) to intestinal bacterial flora after 2 types of inbred (BALB/c) mother mice (a BALB/c mouse ["non-deficient" in the diagram] and a PP-deficient mouse ["PP-deficient" in the diagram]) were allowed to freely drink water containing an antibiotic mixture and inoculated with 3 types of bacterial strains *(Parabacteroides goldsteinii, Bacteroides acidifaciens,* and *Prevotella buccalis*) ("*P. Goldsteinii-*inoculated", "*B. Acidifaciens-inoculated",* and "*P*. Buccalis-inoculated" in the diagram) or were not inoculated with these bacterial strains ("uninoculated" in the diagram). "*", "***", and "****" in the diagram depict statistically significant difference (p < 0.05, p < 0.001, and p < 0.0001, respectively).

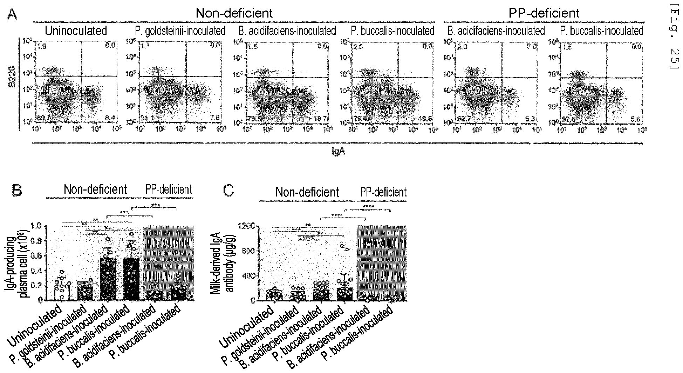

[Figure 25] Figure 25A is a diagram showing results of analyzing plasma cells in the mammary gland with IgA and B220 as markers after 2 types of inbred (BALB/c) mother mice (a BALB/c mouse ["non-deficient" in the diagram] and a PP-deficient mouse ["PP-deficient" in the diagram]) were allowed to freely drink water containing an antibiotic mixture and inoculated with 3 types of bacterial strains *(Parabacteroides goldsteinii, Bacteroides acidifaciens,* and *Prevotella buccalis*) ("*P. Goldsteinii-inoculated",* "*B. Acidifaciens-inoculated",* and "*P*. Buccalis-inoculated" in the diagram) or were not inoculated with these bacterial strains ("uninoculated" in the diagram). Figure 25B is a diagram showing results (mean + standard deviation) of measuring the number of IgA-producing plasma cells (cells included in the lower right area of each graph in Figure 25A) on the basis of the results of Figure 25A. Figure 25C is a diagram showing results (mean + standard deviation) of measuring an IgA antibody concentration in the gastric contents of child mice that ingested the milk of the 2 types of inbred (BALB/c) mother mice inoculated or uninoculated with the 3 types of bacterial strains. "**", "***", and "****" in the diagram depict statistically significant difference (p < 0.01, p < 0.001, and p < 0.0001, respectively).

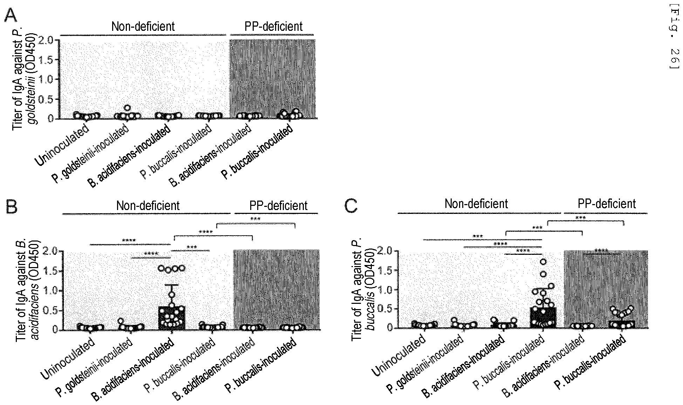

[Figure 26] Figure 26 is a diagram showing results of measuring the titer of an IgA antibody against *Parabacteroides goldsteinii* (Figure 26A), the titer of an IgA antibody *against Bacteroides acidifaciens* (Figure 26B), and the titer of an IgA antibody against *Prevotella buccalis* (Figure 26C) after 2 types of inbred (BALB/c) mother mice (a BALB/c mouse ["non-deficient" in the diagram] and a PP-deficient mouse ["PP-deficient" in the diagram]) were allowed to freely drink water containing an antibiotic mixture and inoculated with 3 types of bacterial strains *(Parabacteroides goldsteinii, Bacteroides acidifaciens,* and *Prevotella buccalis*) ("*P. Goldsteinii-*inoculated", "*B. Acidifaciens-inoculated",* and "*P*. Buccalis-inoculated" in the diagram) or were not inoculated with these bacterial strains ("uninoculated" in the diagram). "***" and "****" in the diagram depict statistically significant difference (p < 0.001 and p < 0.0001, respectively).

### Mode of Carrying Out the Invention

The agent for increasing an IgA antibody content in milk according to the present invention is an agent containing one or more present bacterial strains (i.e., bacterial strains selected from: a bacterial strain of the genus *Bacteroides* which has a 16S rRNA gene having at least 90% identity to the nucleotide sequence of SEQ ID NO: 1, and has an action of increasing an IgA antibody content in milk; and a bacterial strain of the genus *Prevotella* which has a 16S rRNA gene having at least 90% identity to the nucleotide sequence of SEQ ID NO: 2, and has an action of increasing an IgA antibody content in milk), which is identified by the purpose of "increasing an IgA antibody content in milk" (hereinafter, also referred to as the "present increasing agent"). In this context, the 16S rRNA gene is usually contained in the genomic DNA of the present bacterial strain.

For the present increasing agent, the present bacterial strain which is a probiotic having action of increasing an IgA antibody content in milk may be used alone as feed for livestock, a food or drink, or a medicament (preparation), or may be further mixed with an additive and used in the form of a composition (a feed composition for livestock, a food or drink composition or a pharmaceutical composition). Examples of the food or drink can include a health food (a functional food, a nutritional supplement, a health supplement, an engineered food, a nutritionally balanced food, a supplement, etc.) and a food with health claims (a food for specified health use, a food with nutrient function claims, a food with functional claims, etc.). The present increasing agent is preferably feed for livestock or a food or drink.

The present bacterial strain of the genus *Bacteroides* may be a bacterial strain in a live state or a bacterial strain in a killed state as long as the bacterial strain of the genus *Bacteroides* has a 16S rRNA gene having at least 90% identity to the whole nucleotide sequence of SEQ ID NO: 1, and has an action of increasing an IgA antibody content in milk. The present bacterial strain of the genus *Bacteroides* also encompasses a bacterial strain of the genus *Bacteroides* that has a 16S rRNA gene having at least 90% identity to the nucleotide sequence of SEQ ID NO: 1, and has an action of increasing an IgA antibody content in milk, though differing in species from *Bacteroides acidifaciens* whose effect has been demonstrated in the present Examples mentioned later. Examples of the present bacterial strain of the genus *Bacteroides* can include *Bacteroides acidifaciens* (e.g., JCM10556 strain [GenBank accession No: EU136694], NM70_E10 strain [GenBank accession No: MK929079], A40 strain [GenBank accession No: NR_028607], and JJM0207-2 strain [GenBank accession No: KR364740]), *Bacteroides caecimuris* (e.g., I48 strain [GenBank accession No: CP015401] and NM63_1-25 strain [GenBank accession No: MK929076]), *Bacteroides thetaiotaomicron* (e.g., BCRC10624 strain [GenBank accession No: EU136679] and 7330 strain [GenBank accession No: CP012937]), and *Bacteroides ovatus* (e.g., ATCC 8483 strain [GenBank accession No: CP012938] and V975 strain [GenBank accession No: LT622246]).

The present bacterial strain of the genus *Prevotella* may be a bacterial strain in a live state or a bacterial strain in a killed state as long as the bacterial strain of the genus *Prevotella* has a 16S rRNA gene having at least 90% identity to the whole nucleotide sequence represented by SEQ ID NO: 2, and has an action of increasing an IgA antibody content in milk. The present bacterial strain of the genus *Prevotella* also encompasses a bacterial strain of the genus *Prevotella* that has a 16S rRNA gene having at least 90% identity to the nucleotide sequence of SEQ ID NO: 2, and has an action of increasing an IgA antibody content in milk, though differing in species from *Prevotella buccalis* whose effect has been demonstrated in the present Examples mentioned later. Examples of the present bacterial strain of the genus *Prevotella* can include *Prevotella buccalis* (e.g., JCM12246 strain [GenBank accession No: NR_113098], DCW_SL_32A strain [GenBank accession No: MK424033], ATCC 35310 strain [GenBank accession No: NR_044630], SEQ186 strain [GenBank accession No: JN867261]), BV3C7 strain (GenBank accession No: JN809774), and BV3P1 strain (GenBank accession No: JN809764).

The present bacterial strain of the genus *Bacteroides* and the present bacterial strain of the genus *Prevotella* more specifically have action of activating an immune function in Peyer's patches of gut lymphoid tissues, and increasing the number of IgA antibody-producing plasma cells.

The present bacterial strain may be a naturally occurring present bacterial strain of the genus *Bacteroides* and present bacterial strain of the genus *Prevotella,* or may be a modified form of the naturally occurring present bacterial strain of the genus *Bacteroides* and present bacterial strain of the genus *Prevotella* by use of a gene recombination technique or the like. Examples of such a modified strain can include a modified strain expressing a bacterial or viral antigen responsible for infectious disease (e.g., diarrhea) caused in a child at the juvenile stage in order to produce a specific IgA antibody against the antigen into milk, and a modified strain expressing an antigen responsible for infectious disease (e.g., mastitis/garget) caused in the mammary gland in order to induce the production of an IgA antibody against the antigen in the mammary gland of a mammalian mother. The present increasing agent comprising such a modified strain is also useful as a maternal vaccination-type mother-to-baby transfer vaccine or a vaccine targeting the mammary gland (breast) of a mother.

In the present specification, the "increase in IgA antibody content in milk" means that an IgA antibody content in milk in a breast-feeding mammalian mother is increased as compared with an IgA antibody content in milk in a breast-feeding mammalian mother serving as a comparative control (hereinafter, also referred to as a "comparative control mother"). The comparative control mother is not particularly limited and may be a breast-feeding mammalian mother free from a factor that disturbs intestinal bacterial flora, such as stress or the administration of an antibiotic. Preferred examples thereof can include a breast-feeding mammalian mother with a decreased ratio of the present bacterial strain of the genus *Bacteroides* and/or the present bacterial strain of the genus *Prevotella* to the whole intestinal bacterial flora as compared with a breast-feeding mammalian mother free from a factor that disturbs intestinal bacterial flora, such as stress or the administration of an antibiotic (in other words, a mammalian mother having normal intestinal bacterial flora), because its effect has been demonstrated in the present Examples mentioned later. As for whether the IgA antibody content in milk has been increased, an arbitrary threshold (cutoff value) can be set. Examples of such a threshold can include a mean of IgA antibody contents in milk in comparative control mothers; mean + standard deviation (SD) ; mean + 2SD; mean + 3SD; a median value; and an interquartile range. Alternatively, the threshold may be calculated using a ROC (Receiver Operating Characteristic) curve and using statistical analysis software on the basis of data on an IgA antibody content in milk in a breast-feeding mammalian mother inoculated with the present bacterial strain and data on an IgA antibody content in milk in a breast-feeding mammalian mother that is not inoculated with the present bacterial strain in order to enhance sensitivity (ratio at which a breast-feeding mammalian mother inoculated with the present bacterial strain can be correctly determined to be positive) and specificity (ratio at which a breast-feeding mammalian mother that is not inoculated with the present bacterial strain can be correctly determined to be negative).

In the present specification, examples of the mammal can include a human and a nonhuman mammal (e.g., a monkey, a mouse, a rat, a dog, a cat, and livestock [e.g., a rabbit, a pig, a horse, a bovine, sheep, a goat, and a deer]) and can preferably include a human and livestock.

The subject to which the present increasing agent is inoculated (also referred to as "administered" or "applied", depending on the form of the present increasing agent) can be a mammalian mother (preferably a breast-feeding mammalian mother) in need of increase in IgA antibody content in milk. Preferred examples thereof can include a mammalian mother with a decreased ratio of the present bacterial strain of the genus *Bacteroides* and the present bacterial strain of the genus *Prevotella* to the whole intestinal bacterial flora, because its effect has been demonstrated in the present Examples mentioned later.

In the present invention, the "at least 90% identity to the nucleotide sequence represented by SEQ ID NO: 1 (or 2)" means that a sequence derived from the nucleotide sequence of SEQ ID NO: 1 (or 2) by the substitution, deletion, insertion, addition or inversion of one or several nucleotides is identical to 90% or more of the whole nucleotide sequence of SEQ ID NO: 1 (or 2). In this context, the "nucleotide sequence derived by the substitution, deletion, insertion, addition or inversion of one or several nucleotides" means a nucleotide sequence derived by the substitution, deletion, insertion, addition or inversion of the number of nucleotides within the range of 1 to 149, preferably within the range of 1 to 100, more preferably within the range of 1 to 75, further preferably within the range of 1 to 50, more preferably within the range of 1 to 40, further preferably within the range of 1 to 30, more preferably within the range of 1 to 15.

In the present invention, the "at least 90% identity" is preferably 910 or higher, more preferably 92% or higher, further preferably 93% or higher, furthermore preferably 94% or higher, particularly preferably 95% or higher, particularly more preferably 96% or higher, particularly further preferably 97% or higher, particularly furthermore preferably 98% or higher, most preferably 99% or higher (e.g., 99.10 or higher, 99.2% or higher, 99.3% or higher, 99.4% or higher, 99.5% or higher, 99.6% or higher, 99.7% or higher, 99.8% or higher, 99.9% or higher, or 100%) identity. The identity of a nucleotide sequence can be determined through the use of a program called BLASTN (Altschul SF, et al: J Mol Biol 215: 403, 1990) based on a program called BLASTX or BLASTP (Altschul SF, et al: J Mol Biol 215: 403, 1990) based on algorithm BLAST by Karlin and Altschul (Proc. Natl. Acad. Sci. USA 87: 2264-2268, 1990; and Proc Natl Acad Sci USA 90: 5873, 1993). In the case of analyzing a nucleotide sequence using BLASTN, parameters are set to, for example, score = 100 and word length = 12.

The present increasing agent is broadly classified into liquid type and nonliquid type. The present increasing agent of liquid type can be produced by purifying the present bacterial strain from a culture solution of the present bacterial strain, adding thereto appropriate physiological saline or replacement fluid, or pharmaceutical additive, if necessary, and packing an ampule or a vial with the mixture. On the other hand, the present increasing agent of nonliquid type can be produced by adding an appropriate cryoprotectant (e.g., glycerol, dimethyl sulfoxide [DMSO], trehalose, or dextran) to the present increasing agent of liquid type, and packing an ampule or a vial with the mixture, followed by freezing or freeze drying.

The method for inoculating the present increasing agent may be an oral inoculation method or may be a parenteral inoculation method (e.g., intravenous administration or local administration). Preferred examples thereof can include an oral inoculation method, because its effect has been demonstrated in the present Examples mentioned later.

In the present specification, examples of the additive can include a pharmaceutically acceptable usual formulation ingredient such as a carrier, a binder, a stabilizer, an excipient, a diluent, a pH-buffering agent, a disintegrant, a tonicity agent, an additive, a coating agent, a solubilizer, a lubricant, a sliding agent, a dissolution aid, a lubricating agent, a flavoring agent, a sweetener, a solvent, a gelling agent, and a nutrient. Examples of such a formulation ingredient can specifically include water, physiological saline, animal fat and oil, plant oil, lactose, starch, gelatin, crystalline cellulose, gum, talc, magnesium stearate, hydroxypropylcellulose, polyalkylene glycol, polyvinyl alcohol, and glycerin.

The inoculum dose of the present bacterial strain contained in the present increasing agent differs depending on the organism species, age, body weight, physical condition, etc. of the subject (mammalian mother) to be inoculated and therefore, cannot be generalized. The inoculum dose is, for example, 10⁴ to 10¹² cfu (colony forming unit), preferably 10⁶ to 10¹⁰ cfu, per kg of body weight per day. Such an amount may be inoculated in one portion or may be inoculated in several divided portions. When the present increasing agent is a feed composition for livestock, the amount of the present bacterial strain contained in the feed composition for livestock is, for example, 10⁴ to 10¹² cfu/g, preferably 10⁶ to 10¹⁰ cfu, per g of the feed composition for livestock.

Hereinafter, the present invention will be described more specifically with reference to Examples. However, the technical scope of the present invention is not limited by these examples.

### Examples

### 1. Material and method

### [Bacterial strain]

3 types of bacterial strains (*Parabacteroides goldsteinii* [JCM13446], *Bacteroides acidifaciens* [JCM10556], and *Prevotella buccalis* [JCM12246]) were obtained from the Microbe Division of the RIKEN BioResource Research Center. These bacteria were each cultured overnight in GAM liquid medium under anaerobic conditions using a bacterial culture gas concentration adjuster (Anaeropack[R]) (manufactured by SUGIYAMA-GEN Co., Ltd.), followed by bacterial counting.

### [Model animal]

6 types of mice (BALB/c[BALB/cCrSlc] mice; C57BL/6[C57BL/6NCrSlc] mice; ICR[C.B-17/Icr-+/+Jcl] mice; ICR mice having severe combined immunodeficiency disease [C.B-17/Icr-scid/scidJcl] (i.e., immunodeficient mice); and B6N.Cg-Tg[Vil-cre]997Gum/J mice) were purchased from Japan SLC, Inc., CLEA Japan, Inc., The Jackson Laboratory, or Sankyo Labo Service Corporation, Inc. and raised in the animal facility of the Tohoku University Graduate School of Agricultural Science or the Institute Of Medical Science, the University Of Tokyo. B6-Tg(CAG-FLPe)36 mice were kindly provided by the RIKEN BioResource Research Center. PP-deficient mice used in the experiments of Figures 2 and 23 to 26 were prepared by administering 1 mg of an anti-IL-7Rα antibody (A7R34) to the uterus of BALB/c mice on the 14th day of intrauterine life (see the document "Yoshida et al., International Immunology, 11, 643-655. 1999"). ILN-deficient mice used in the experiments of Figure 2 were prepared by surgically removing inguinal lymph node. Mice used in the experiments of Figure 3 were prepared by administering PP-derived mononuclear cells and ILN-derived mononuclear cells obtained in accordance with the methods described in the section [Isolation of cell] given below to the tail vein of wild-type (Icr^{+/+}) mice at the start of mating of immunodeficient mice. Mice used in the experiments of Figures 5 to 7 were prepared by allowing BALB/c mother mice to freely drink water containing an antibiotic(1 g/L ampicillin, 1 g/L neomycin, 500 mg/L vancomycin, or a mixture thereof) for a period from before mating to 14 days after birth. Mice used in the experiments of Figures 8 to 10 and 16 to 18 were prepared by allowing BALB/c mother mice to freely drink water containing an antibiotic mixture for a period from before mating to 7 days after birth, and then performing FMT a total of 7 times (once a day) for a period from 8 to 14 days after birth using stool collected from healthy BALB/c mice (Figures 8 to 10 and 16 to 18) or stool collected from immunodeficient mice (Figures 16 to 18). Bone marrow transplantation (BMT) in mice used in the experiments of Figures 12 to 15 was performed using 10⁶ bone marrow mononuclear cells obtained from wild-type (Icr^{+/+}) mice at the start of mating of immunodeficient mice. Mice used in the experiments of Figures 20 to 22 were prepared by allowing BALB/c mother mice to freely drink water containing an antibiotic mixture for a period from before mating to 7 days after birth, and then orally administering 500 µL of PBS containing approximately 10¹⁰ cfu of 3 types of bacterial strains *(Parabacteroides goldsteinii, Bacteroides acidifaciens,* and *Prevotella buccalis).* All the animal experiments were designed in accordance with a protocol approved by the Institutional Animal Care and Use Committees of both the Tohoku University and the Tokyo University.

### [Preparation of SpiB cKO mouse]

A targeting vector was designed such that exons 2 to 5 of Spib gene were flanked by loxP sites and an Frt-flanked neo resistance cassette was inserted to an intron between exons 5 and 6. After linearization of 30 µg of the targeting vector, JM8A1.N3 embryonic stem cells (obtained from the KOMP Repository) were transformed therewith. Colonies of neomycin-resistant and ganciclovir-resistant embryonic stem cells were selected and then screened for homologous recombinants targeted for the Spib gene by PCR using primer sets shown in Table 1 below and Southern blot analysis using probes shown in Table 1 below. The homologous recombinants thus obtained by screening were microinjected to the blastocysts of C57BL/6 mice to prepare chimeric mice. In order to obtain floxed Spib mice by the deletion of the Neo resistance cassette, heterozygous F1 mice were mated with B6-Tg(CAG-FLPe)36 mice. Next, the obtained floxed Spib mice were mated with B6N.Cg-Tg(Vil-cre)997Gum/J mice to prepare Spib^{flox/flox} mice. The prepared Spib^{flox/flox} mice were mated with mice expressing Cre recombinase in a gut epithelial cell-specific manner (Villin-Cre transgenic mice) to prepare SpiB conditional knockout mice (i.e., SpiB cKO mice).

**[Table 1]**

| Primer | Base sequence |
|---|---|
| Primer set for distal introduced loxP detection | |
| Primer 1 | 5' -TAGTTCATTGCTGCTCCTAGTCTCAGC-3' |
| Primer 2 | 5' -TGGGATTTGCATGCACAGACGCTGGGG-3' |
| Primer 4 for Neo insertion detection | |
| Primer 4 | 5' -CTAAAGCGCATGCTCCAGACTGCCTTG-3' |
| Primer 5 | 5' -GAGGCAAGAGGACTGCCACTAGCTTGAG-3' |
| Primer set for proximal introduced IoxP and Neo deletion detection | |
| Primer 3 | 5' -CTTATTTATACTCCCGCGCTCACTGGAGG-3' |
| Primer 5 | 5' -GAGGCAAGAGGACTGCCACTAGCTTGAG-3' |
| Probe for Southern blot | |
| Forward probe | 5' -GGTAGACAGACTTCTAGACTTGGTCAACCC-3' |
| Reverse probe | 5' -GTTTAGCCTTGACCACATGGACATCTGG-3' |

### [Isolation of cell]

Mononuclear cells were isolated from the mammary gland, PP, and ILN of each mouse. Specifically, the mammary gland and PP were digested with 0.5 mg/mL collagenase at 37°C for 60 minutes to isolate mammary gland-derived mononuclear cells and PP-derived mononuclear cells. ILN-derived mononuclear cells were prepared by physically treating ILN. Some of the obtained mononuclear cells were stained with Turk's solution and, after cell counting, used in flow cytometry and administration into the tail vein.

### [Flow cytometry]

The mononuclear cells isolated from the respective tissues were blocked with 10 pg/mL anti-mouse CD16/32 (2.4G2) at 4°C for 15 minutes. Antigen-antibody reaction treatment was performed at 4°C for 30 minutes in the presence of 5 types of antibodies against 5 types of cell surface markers (B220, Ly6C, CD93, I-Ad, and CD11b) labeled with a fluorescent material (2 pg/mL BV421-labeled anti-CD45R [B220] antibody [RA3-6B2, manufactured by BD Biosciences], 2 pg/mL PE-labeled anti-Ly6C antibody [HK1.4, manufactured by BioLegend, Inc.], 2 pg/mL PE-Cy7-labeled anti-CD93 antibody [AA4.1, manufactured by BioLegend, Inc.], 5 pg/mL Alexa 647-labeled anti-I-Ad antibody [39-10-8, manufactured by BioLegend, Inc.], and 2 ug/mL BV510-labeled anti-CD11b antibody [M1/70, manufactured by BD Biosciences]). The isotype control antibodies used were 4 types of antibodies (5 µg/mL Alexa 647-labeled mouse IgG3 antibody [MG3-35, manufactured by BioLegend, Inc.], 2 pg/mL BV421-labeled rat IgG2a [R35-95, manufactured by BD Biosciences], 2 pg/mL PE-Cy7-labeled rat IgG2b [RTK4530, manufactured by BioLegend, Inc.], and 2 ug/mL PE-labeled rat IgG2c [RTK4174, manufactured by BioLegend, Inc.]). In order to remove dead cells in flow cytometry analysis, Cell Viability Solution (manufactured by BD Biosciences) was added at 10 pg/test. In order to detect an IgA antibody in cells, the mononuclear cells isolated from the respective tissues were fixed in a 4% (w/v) paraformaldehyde solution at room temperature for 20 minutes and treated with a surfactant (0.1% [w/v] saponin) at room temperature for 15 minutes, followed by antigen-antibody reaction treatment at room temperature for 30 minutes in the presence of 5 pg/mL FITC-labeled anti-IgA antibody (C10-3, manufactured by BD Biosciences). The isotype control antibodies used were 2 types of antibodies (5 pg/mL FITC rat IgG1 [R3-34, manufactured by BD Biosciences] and 2 ug/mL BV421 rat IgG1 [R3-34, manufactured by BD Biosciences]). Flow cytometry was performed using Attune NxT Acoustic Focusing Cytometer (manufactured by Thermo Fisher Scientific, Inc.) or Accuri C6 flow cytometer (manufactured by BD Biosciences).

### [ELISA]

In order to measure the concentration of an IgA antibody in milk in mother mice, the concentration of an IgA antibody in gastric contents collected from child mice that ingested the milk (hereinafter, also referred to as a "milk-derived IgA antibody") was measured by use of ELISA. Specifically, the gastric contents of the child mice that ingested the milk were suspended in 10 µL of PBS per mg, and a supernatant (hereinafter, referred to as a "milk-derived sample") was recovered by centrifugation. Next, 100 ug/mL anti-IgA antibody (manufactured by Bethyl Laboratories, Inc.) was added to each well of a 96-well ELISA plate and incubated overnight at 4°C for immobilization treatment. After blocking treatment at room temperature for 1 hour in the presence of 1% (w/v) BSA, the milk-derived sample in two serial dilutions was added to each well and incubated at room temperature for 2 hours. After washing with PBS, 100 ng/mL HRP-labeled IgA antibody (anti-IgA antibody differing in epitope from the immobilized anti-IgA antibody) (manufactured by Bethyl Laboratories, Inc.) was added to each well and incubated at room temperature for 1 hour. HRP-derived signals were generated using TMB (tetramethylbenzidine) microwell peroxidase substrate system (manufactured by SeraCare Life Sciences Inc.). The calibration curve of the IgA antibody was prepared using mouse serum containing a known concentration of the IgA antibody. An IgA antibody concentration in the gastric contents of the child mice that ingested the milk was measured on the basis of the calibration curve.

The milk-derived IgA antibody was analyzed for its reactivity with stool (containing intestinal microbes) or 3 types of bacterial strains (*Parabacteroides goldsteinii, Bacteroides acidifaciens,* and *Prevotella buccalis*) by use of ELISA. Specifically, PBS containing 100 pg/mL stool and PBS containing 10 µg/mL each of the 3 types of bacterial strains were each filtered through a cell strainer with a pore size of 100 µm, added to each well of a 96-well ELISA plate, and incubated overnight at 4°C for immobilization treatment. The plate in which the stool was immobilized was blocked at room temperature for 1 hour in the presence of 1% (w/v) BSA and 1 µg/mL anti-IgA antibody, and the plates in which the 3 types of bacterial strains were immobilized were blocked at room temperature for 1 hour in the presence of 1% (w/v) BSA. Then, the milk-derived sample diluted at 1:64 or the undiluted milk-derived sample was added to each well and incubated at room temperature for 2 hours. After washing with PBS, 100 ng/mL HRP-labeled IgA antibody (manufactured by Bethyl Laboratories, Inc.) was added to each well and incubated at room temperature for 1 hour. HRP-derived signals were generated using TMB microwell peroxidase substrate system (manufactured by SeraCare Life Sciences Inc.). The titer of milk IgA against the stool (containing intestinal microbes) or the 3 types of bacterial strains was calculated as an OD₄₅₀ value.

### [IgA-Seq]

In order to identify an intestinal bacterial species to which an IgA antibody in milk bound, a partially revised method of IgA-Seq described in the document "Palm et al., Cell 158, 1000-1010. 2014" was performed. Specifically, debris was removed using a cell strainer with a pore size of 40 µm from PBS (100 µg/µL) containing stool collected from a healthy BALB/c mouse to prepare a stool suspension containing intestinal microbes. After blocking treatment at 4°C for 30 minutes in the presence of 500 µg/mL anti-IgA antibody, 20% (v/v) normal rat serum, and 1% (w/v) BSA, the stool suspension was mixed with the milk-derived sample at a ratio of 1:1 and incubated at 4°C for 30 minutes. After washing, the suspension was incubated at 4°C for 30 minutes in the presence of 2 ug/mL PE-labeled anti-IgA antibody (mA-6E1, manufactured by Invitrogen Corp.) and a 500 nM cellular nucleus staining reagent (SYTO 9) and then incubated at 4°C for 30 minutes in the presence of Anti-PE MicroBeads UltraPure (manufactured by Miltenyi Biotec) at 30 pL/test. A bacterium to which an IgA antibody bound was recovered using a magnetic cell sorter AutoMACS (manufactured by Miltenyi Biotec), and bacterium-derived genomic DNA was extracted using Stool DNA Isolation Kit (manufactured by Chiyoda Science Co., Ltd.). The intestinal bacterial species to which an IgA antibody in milk bound was identified in accordance with the method described in the section [Metagenomic analysis] given below with the genomic DNA as a template.

### [Metagenomic analysis]

Bacterium-derived genomic DNA was extracted from the stool of each mouse using QIAamp DNA Stool Mini Kit (manufactured by Qiagen N.V.), and metagenomic analysis was conducted. Specifically, the V3 and V4 regions of 16S rRNA gene of each bacterium were amplified by PCR with the extracted bacterium-derived genomic DNA as a template using PrimeSTAR HS DNA polymerase (manufactured by Takara Bio Inc.) and primers shown in Table 2 below.

**[Table 2]**

| Forward primer mixture | |
|---|---|
| No. 1 | 5' -TGCTCTTCCGATCTGACNNNCCTACGGGNGGCWGCAG-3' |
| No. 2 | 5' -TGCTCTTCCGATCTGACNNNNCCTACGGGNGGCWGCAG-3' |
| No. 3 | 5' -TGCTCTTCCGATCTGACNNNNNCCTACGGGNGGCWGCAG-3' |
| No. 4 | 5' -TGCTCTTCCGATCTGACNNNNNNCCTACGGGNGGCWGCAG-3' |

| Reverse primer mixture | |
|---|---|
| No. 1 | 5' -CGCTCTTCCGATCTCTGNNNGACTACHVGGGTATCTAATCC-3' |
| No. 2 | 5' -CGCTCTTCCGATCTCTGNNNNGACTACHVGGGTATCTAATCC-3' |
| No. 3 | 5' -CGCTCTTCCGATCTCTGNNNNNGACTACHVGGGTATCTAATCC-3' |
| No. 4 | 5' -CGCTCTTCCGATCTCTGNNNNNNGACTACHVGGGTATCTAATCC-3' |

In the table, the single underlined portions depict adaptor tag sequences, and the double underlined portions depict spacer sequences.

The PCR amplification products obtained by the first PCR were subjected to the second PCR using a forward primer for the second PCR containing a 6-base index represented by "xxxxxx" (5'-CAAGCAGAAGACGGCATACGAGATxxxxxxGTGACTGGAGTTCAGACGTGTGCTCTTC CGATCTGAC-3') and a reverse primer for the second PCR (5'-AATGATACGGCGACCACCGAGATCTACACTCTTTCCCTACACGACGCTCTTCCGATCT CTG-3'), as described in the document "Palm et al., Cell 158, 1000-1010. 2014", in order to discriminate among the respective samples. The obtained PCR amplification products were sequenced using MiSeq platform and MiSeq reagent kit v. 2 (manufactured by Illumina, Inc.). The obtained data was analyzed using BaseSpace (manufactured by Illumina, Inc.) to identify a bacterial species.

### [Quantitative PCR]

Quantitative PCR was performed using TB Green Premix Ex Taq II (manufactured by Takara Bio Inc.) with the bacterium-derived genomic DNA, which was extracted in accordance with the method described in the section [IgA-Seq] or [Metagenomic analysis], as a template to quantify the copy number of bacterium-specific tuf gene. All primers were designed with Perfect Real-time support system (manufactured by Takara Bio Inc.).

### [Tissue staining]

Small intestinal tissues were isolated in accordance with a standard method from mice that underwent BMT, fixed in a 4% (w/v) paraformaldehyde solution, and then embedded in paraffin. Tissue sections (5 pm) were blocked in a TNB solution at room temperature for 30 minutes, followed by antigen-antibody reaction treatment overnight at 4°C in the presence of PE-labeled anti-CD45R (B220) antibody (RA3-6B2) and anti-human CD3 (SP2) antibody cross-reacting with mouse CD3. After washing, the tissue sections were incubated at room temperature for 1 hour in the presence of HRP-labeled IgG antibody. CD3-derived signals were amplified at room temperature for 10 minutes using TSA Plus fluorescein System (manufactured by PerkinElmer, Inc.). The cellular nuclei were stained using 1 pg/mL DAPI. For histopathological analysis, the tissue sections were stained with hematoxylin-eosin, and images were obtained using BZ-9000 (manufactured by Keyence Corp.) or BX63 (manufactured by Olympus Corp.).

### 2. Results

### [Expression analysis of cell surface marker in IgA antibody-producing plasma cell]

It has been reported that the migration of IgA antibody-producing plasma cells into the mammary gland is essential for producing IgA in the mammary gland (see the documents "Halsey et al., Ann N Y Acad Sci 409, 452-460. 1983" and "Niimi et al., Mucosal Immunol 11, 643-653. 2018"). Accordingly, in order to identify IgA-producing plasma cells in the mammary gland of mother mice, flow cytometry was performed using 6 types of plasma cell-related markers (IgA antibody and 5 types of cell surface markers [B220, Ly6C, I-Ad, CD11b, and CD93]). As a result, plasma cells producing a high level of an IgA antibody were found positive to 3 types of cell surface markers (Ly6C, I-Ad, and CD93) and negative to 2 types of cell surface markers (B220 and CD11b) (see Figure 1). Hence, in the subsequent experiments, B220-negative and IgA-positive plasma cells were used as an index for IgA-producing plasma cells.

### [Peyer's patches play major role in mother IgA antibody production in mammary gland]

Inguinal lymph node (ILN) is known to play a role as the draining lymph node of the mammary gland (see the document "Leonhardt, Gene 94, 121-124. 1990"). Also, Peyer's patches (PP) are known to play a very important role in the mucosal immune system in the gastrointestinal tract in which, particularly, an IgA antibody is most abundantly produced (see the documents "Lindner et al., Nat Immunol 16, 880-888. 2015" and "Moro-Sibilot et al., Gastroenterology 151, 311-323. 2016"). Accordingly, influence on mother IgA antibody production in the mammary gland was analyzed using 2 types of model mother mice (ILN-deficient mice and PP-deficient mice). As a result, the ratio and number of IgA-producing plasma cells in the mammary gland of the ILN-deficient mice were rarely different from the ratio and number of IgA-producing plasma cells in the mammary gland of the non-deficient mice, whereas the ratio and numeric value of IgA-producing plasma cells in the mammary gland of the PP-deficient mice were found significantly lower than the ratio and numeric value of IgA-producing plasma cells in the mammary gland of the non-deficient mice (see Figures 2A and 2B). The concentration of a milk-derived IgA antibody in the ILN-deficient mice was rarely different from the concentration of a milk-derived IgA antibody in the non-deficient mice, whereas the concentration of a milk-derived IgA antibody in the PP-deficient mice was found significantly lower than the concentration of a milk-derived IgA antibody in the non-deficient mice (see Figure 2C).

Even when wild-type (Icr^{+/+}) mouse ILN-derived mononuclear cells were transferred to immunodeficient (C.B-17/Icr-scid/scidJcl) mother mice deficient in T cells and B cells, the ratio and number of IgA-producing plasma cells in the mammary gland and a milk-derived IgA antibody concentration were rarely different from those of immunodeficient mother mice without transfer. By contrast, when wild-type mouse PP-derived mononuclear cells were transferred to immunodeficient mother mice, the ratio and numeric value of IgA-producing plasma cells in the mammary gland and a milk-derived IgA antibody concentration were found significantly higher than those of immunodeficient mother mice without transfer (see Figure 3).

These results indicate that PP (not ILN) is essential for the migration of IgA-producing plasma cells into the mammary gland.

### [Antigen uptake of M cell in PP is necessary for mother IgA antibody production in mammary gland]

Spib is known as a transcriptional factor involved in the differentiation of M cells which take up an antigen present in follicle-associated epithelium (FAE) into mature B cells producing an IgA antibody in the gut epithelium containing FAE (see the documents "Kanaya et al., Nat Immunol 13, 729-736. 2012" and "Sato et al., Mucosal Immunol 6, 838-846. 2013"). Meanwhile, Spib gene conditional knockout model mice (i.e., SpiB cKO mice) are mice deficient in M cells in Peyer's patches (PP). Accordingly, the relation of Spib to IgA production in the mammary gland was analyzed using the SpiB cKO mice. As a result, the ratio and numeric value of IgA-producing plasma cells in the mammary gland of the SpiB cKO mice were found significantly lower than the ratio and numeric value of IgA-producing plasma cells in the mammary gland of Spib^{flox/flox} mice (i.e., non-SpiB gene-deficient mice) (see Figures 4A and 4B). The concentration of a milk-derived IgA antibody in the SpiB cKO mice was found significantly lower than the concentration of a milk-derived IgA antibody in Spib^{flox/flox} mice (see Figure 4C).

These results indicate that Spib is involved in IgA production in the mammary gland. These results together with the results of Figures 2 and 3 taken into consideration indicate that an antigen in the gut taken up by M cells activates an immune function in PP and thereby increases the number of IgA-producing plasma cells, and the IgA-producing plasma cells increased in number migrate into the mammary gland, resulting in an increased IgA antibody content in milk.

### [Intestinal microbe promotes IgA antibody production in milk]

In consideration of the fact that intestinal bacterial flora establishes homeostasis together with host immunocytes in the gastrointestinal tract containing PP, influence on mother IgA antibody production was examined by administering various types of antibiotics, specifically, ampicillin, neomycin, or vancomycin, or a mixture thereof to BALB/c mother mice for a period from pregnancy to breast-feeding, and thereby disturbing intestinal bacterial flora. First, in order to examine the influence of each antibiotic treatment on intestinal bacterial flora, metagenomic analysis was conducted. As a result, the total number of intestinal bacteria was rarely different between each antibiotic treatment and no treatment (see Figure 5A), whereas the constitution of the intestinal bacterial flora drastically varied therebetween (see Figures 5B and 5C). Particularly, the ratios of 4 types of bacterial strains *(Parabacteroides goldsteinii, Bacteroides acidifaciens, Prevotella buccalis,* and *Escherichia albertii*) to the intestinal bacterial flora largely varied (see Figure 6). Specifically, when vancomycin or a mixture consisting of 3 types of antibiotics (ampicillin, neomycin, and vancomycin) was administered to mother mice by free drinking, the ratios of 3 types of bacterial strains (*Parabacteroides goldsteinii* [Figure 6A], *Bacteroides acidifaciens* [Figure 6B], and *Prevotella buccalis* [Figure 6C]) to the intestinal bacterial flora were drastically decreased as compared with mother mice given no antibiotic, whereas the ratio of 1 type of bacterial strain (*Escherichia albertii* [Figure 6D]) was higher. Also, when vancomycin or a mixture consisting of the 3 types of antibiotics was administered to mother mice by free drinking, the ratio and numeric value of IgA-producing plasma cells in the mammary gland of the mother mice were significantly lower than those of mother mice given no antibiotic (see Figures 7A and 7B) and the concentration of a milk-derived IgA antibody was also found significantly lower (see Figure 7C).

These results indicate that any of 3 types of bacterial strains (*Parabacteroides goldsteinii, Bacteroides acidifaciens,* and *Prevotella buccalis)* contained in the intestinal bacterial flora of a mammalian mother are involved in IgA antibody production in the mammary gland.

In order to test more detailed relation of the 3 types of bacterial strains in the intestinal bacterial flora to IgA antibody production in the mammary gland, a mixture consisting of the 3 types of antibiotics was administered to mother mice to disturb intestinal bacterial flora, followed by FMT using stool collected from healthy BALB/c mice (in which the 3 types of bacterial strains lived symbiotically). As a result, slight increase in the total number of intestinal bacteria in the mother mice that underwent FMT was confirmed as compared with mother mice that did not undergo FMT (see Figure 8A), whereas the ratios of bacteria constituting the intestinal bacterial flora drastically varied therebetween (see Figures 8B and 8C). The ratios of 4 types of bacterial strains (*Parabacteroides goldsteinii, Bacteroides acidifaciens, Prevotella buccalis,* and *Escherichia albertii*), particularly, 2 types of bacterial strains (*Bacteroides acidifaciens* and *Prevotella buccalis*), to the intestinal bacterial flora were found significantly higher in the mother mice that underwent FMT than in mother mice that did not undergo FMT (see Figure 9). In the mother mice that underwent FMT, the ratio and numeric value of IgA-producing plasma cells in the mammary gland were significantly higher than those of mother mice that did not undergo FMT (see Figures 10A and 10B) while a milk-derived IgA antibody concentration was also found significantly higher (see Figure 10C).

In order to analyze the relation of IgA antibody production in milk to intestinal bacterial flora, GF (germ free) mice having no intestinal bacterial flora and SPF (special pathogen-free) mice having physiological intestinal bacterial flora were analyzed for a milk-derived IgA antibody concentration.

Milk samples were collected from mice during lactation maintained in a SPF facility or a germ-free facility. The milk-derived IgA antibody concentration of the GF mice was significantly lower than the milk-derived IgA antibody concentration of the SPF mice and was rather found close to the milk-derived IgA antibody concentration of immunodeficient mice lacking all subclasses of antibodies (see Figure 11A). The milk-derived IgA antibody from the SPF mice exhibited reactivity with microbes contained in the stool of the SPF mice and however, rarely exhibited reactivity with microbes contained in the stool of immunodeficient mice (see Figure 11B). The milk-derived IgA antibody from the SPF mice was confirmed to not react with the stool of the microbe-free GF mice used as a control. These results suggested the possibility that host immunocytes, particularly, lymphocytes, create an intestinal microbial environment involved in IgA antibody production in milk. Thus, analysis was conducted using immunodeficient mice.

Few Peyer's patches were observed in the immunodeficient mouse, whereas a PP-like lymph structure rich in B cells and T cells was confirmed by the transplantation of wild-type mouse bone marrow-derived mononuclear cells to the immunodeficient mice (BMT) (see Figure 12).

These results indicate that PP-like gut lymphoid tissues were formed as a result of reconstruction of the immune system by transplanting wild-type mouse bone marrow-derived mononuclear cells to the immunodeficient mice after birth, though the organogenesis of PP has been reported to start before birth (see the document "Honda et al., J Exp Med 193, 621-630. 2001").

Although the total number of intestinal bacteria was rarely different between the immunodeficient mice in which wild-type mouse bone marrow-derived mononuclear cells were transplanted and the immunodeficient mice without transplantation (see Figure 13A), the constitution of the intestinal bacterial flora drastically varied by transplanting wild-type mouse bone marrow-derived mononuclear cells (see Figures 13B and 13C). The milk-derived IgA antibody from wild-type mice exhibited reactivity not only with microbes contained in the stool of the wild-type mice but with microbes contained in the stool of the immunodeficient mice in which wild-type mouse bone marrow-derived mononuclear cells were transplanted (see Figure 14A). On the other hand, the reactivity of immunodeficient mouse milk containing no IgA antibody with microbes contained in the stool of wild-type mice, or the reactivity thereof with microbes contained in the stool of the immunodeficient mice in which wild-type mouse bone marrow-derived mononuclear cells were transplanted was at a background level (see Figure 14B). Furthermore, the ratios of 2 types of bacterial strains (*Bacteroides acidifaciens* and *Prevotella buccalis*) to the intestinal bacterial flora of the immunodeficient mice were significantly increased by transplanting wild-type mouse bone marrow-derived mononuclear cells (see Figure 15).

These results indicate that the immune system of a breast-feeding mother mouse is necessary for 2 types of bacterial strains (*Bacteroides acidifaciens* and *Prevotella buccalis*) to gain a foothold in the intestine of the breast-feeding mother mouse and increase an IgA antibody content in milk.

Further, a mixture consisting of the 3 types of antibiotics was administered to mother mice, and FMT was performed using stool collected from healthy mice in which the 3 types of bacterial strains lived symbiotically, or stool collected from immunodeficient mice in which these bacteria did not live symbiotically. As a result, almost no change in the total number of intestinal bacteria was confirmed between the case of performing FMT using stool collected from healthy mice and the case of performing FMT using stool collected from immunodeficient mice (see Figure 16A), whereas the constitution of the intestinal bacterial flora varied therebetween (see Figures 16B and 16C). Particularly, the ratios of 2 types of bacterial strains (*Bacteroides acidifaciens* and *Prevotella buccalis*) to the intestinal bacterial flora were found significantly higher in the case of performing FMT using the stool of wild-type mice than in the case of performing FMT using the stool of immunodeficient mice (see Figure 17). The ratio and numeric value of IgA-producing plasma cells in the mammary gland and a milk-derived IgA antibody concentration were significantly higher in the case of performing FMT using the stool of wild-type mice than in the case of performing FMT using the stool of immunodeficient mice (see Figure 18).

These results indicate that the immune system of a mammalian mother regulates an intestinal environment with 2 types of bacterial strains (*Bacteroides acidifaciens* and *Prevotella buccalis*) and produces an IgA antibody in the mammary gland.

*Bacteroides acidifaciens* is a bacterial strain of the genus *Bacteroides* having 16S rRNA gene consisting of the nucleotide sequence of SEQ ID NO: 1. *Prevotella buccalis* is a bacterial strain of the genus *Prevotella* having 16S rRNA gene consisting of the nucleotide sequence of SEQ ID NO: 2.

### [Specificity of IgA antibody in milk depends on intestinal microbial environment]

Next, in order to analyze the specificity of an IgA antibody in milk for 2 types of bacterial strains (*Bacteroides acidifaciens* and *Prevotella buccalis*), IgA-Seq was performed using stool collected from healthy BALB/c mice, and the gastric contents of child mice that ingested the milk of BALB/c mother mice. As a result, the 2 types of bacterial strains exhibited a larger ratio of a bacterial strain that bound to the IgA antibody in milk than the ratio of a bacterial strain that did not bind to the IgA antibody in milk (see Figure 19).

These results indicate that the 2 types of bacterial strains (*Bacteroides acidifaciens* and *Prevotella buccalis*) not only promote the entire mother IgA antibody production in the mammary gland but have action of promoting IgA antibody production against these bacterial strains by stimulating immunocytes in PP.

### [IgA antibody production in milk is increased by oral inoculation of B. acidifaciens and P. buccalis]

Whether IgA antibody production in milk was increased was tested by inoculating the 2 types of bacterial strains (*Bacteroides acidifaciens* and *Prevotella buccalis*) to BALB/c mother mice given an antibiotic mixture. The control used was *Parabacteroides goldsteinii.* As a result, in the case of inoculating these bacterial strains, the total number of intestinal bacteria was rarely different from that in the case of not inoculating the bacterial strains (see Figure 20A), and the constitution of the intestinal bacterial flora was confirmed to vary (see Figures 20B and 20C). By contrast, in the case of inoculating the 2 types of bacterial strains (*Bacteroides acidifaciens* and *Prevotella buccalis*), the ratio and number of IgA-producing plasma cells in the mammary gland and a milk-derived IgA antibody concentration were significantly increased as compared with the case of not inoculating the bacterial strains (see Figure 21). On the other hand, in the case of inoculating the control *Parabacteroides goldsteinii,* the ratio and number of IgA-producing plasma cells in the mammary gland and a milk-derived IgA antibody concentration were rarely different from those in the case of not inoculating the bacterial strain (see Figure 21). In the case of inoculating the 2 types of bacterial strains *(Bacteroides acidifaciens* and *Prevotella buccalis*), the amounts of milk-derived IgA antibodies specific for these bacterial strains were found to be significantly increased (see Figure 22).

These results indicate that the inoculation of 2 types of bacterial strains (*Bacteroides acidifaciens* and *Prevotella buccalis*) to a mammalian mother in a state having a decreased amount of an IgA antibody produced in the mammary gland due to disturbed intestinal bacterial flora activates an immune function in PP and increases the amount of an IgA antibody produced in the mammary gland.

### [PP is essential for increase in IgA antibody production in milk by oral inoculation of B. acidifaciens and P. buccalis]

Whether PP was essential for the increase in the amount of an IgA antibody produced in the mammary gland, which was confirmed when 2 types of bacterial strains (*Bacteroides acidifaciens* and *Prevotella buccalis*) were inoculated to mammalian mothers in a state having a decreased amount of an IgA antibody produced in the mammary gland due to disturbed intestinal bacterial flora, was tested using PP-deficient mice. The control used was *Parabacteroides goldsteinii.* As a result, when the 2 types of bacterial strains (*Bacteroides acidifaciens* and *Prevotella buccalis*) were inoculated to non-PP-deficient mice (BALB/c mother mice) and PP-deficient mice by the inoculation of each of these bacterial strains, the total number of intestinal bacteria was rarely different therebetween (see Figure 23A). By contrast, in the case of inoculating *B. acidifaciens,* the ratio of *B. acidifaciens* to the intestinal bacterial flora was significantly increased in both the mice (see Figures 23B, 23C and 24). When the 2 types of bacterial strains were inoculated to non-PP-deficient mice, the ratio and number of IgA-producing plasma cells in the mammary gland and a milk-derived IgA antibody concentration were significantly increased as compared with the case of not inoculating the bacterial strains. By contrast, even when the 2 types of bacterial strains were inoculated to PP-deficient mice, neither increase in the ratio and number of IgA-producing plasma cells in the mammary gland nor increase in milk-derived IgA antibody concentration was confirmed and these numerical values were at the same level as those in the case of inoculating the control *Parabacteroides goldsteinii* to non-PP-deficient mice (see Figure 25). When the 2 types of bacterial strains were inoculated to non-PP-deficient mice, the amounts of milk-derived IgA antibodies specific for these bacterial strains were significantly increased. By contrast, even when the 2 types of bacterial strains were inoculated to PP-deficient mice, no increase in the amounts of milk-derived IgA antibodies specific for these bacterial strains was confirmed (see Figure 26).

These results indicate that PP (specifically, the activation of an immune function in PP) is essential for the increase in the amount of an IgA antibody produced in the mammary gland, which was confirmed when 2 types of bacterial strains (*Bacteroides acidifaciens* and *Prevotella buccalis*) were inoculated to mammalian mothers in a state having a decreased amount of an IgA antibody produced in the mammary gland due to disturbed intestinal bacterial flora.

### Industrial Applicability

The present invention contributes to improvement in productivity in the livestock industry and the prevention of pathogenic bacterial infection or viral infection of infants.

## Claims

1. An agent for increasing an IgA antibody content in milk, comprising one or more bacterial strains selected from:
a bacterial strain of the genus *Bacteroides* which has a 16S rRNA gene having at least 90% identity to the nucleotide sequence represented by SEQ ID NO: 1, and has an action of increasing an IgA antibody content in milk; and
a bacterial strain of the genus *Prevotella* which has a 16S rRNA gene having at least 90% identity to the nucleotide sequence represented by SEQ ID NO: 2, and has an action of increasing an IgA antibody content in milk.

2. The agent according to claim 1, wherein the bacterial strain has an action of activating immune function in Peyer's patches, and increasing the number of IgA antibodyproducing plasma cells.

3. The agent according to claim 1 or 2, wherein the increasing agent is inoculated to a mammalian mother in which a ratio of the bacterial strain of the genus *Bacteroides* having a 16S rRNA gene having at least 90% identity to the nucleotide sequence represented by SEQ ID NO: 1, and the bacterial strain of the genus *Prevotella* having a 16S rRNA gene having at least 90% identity to the nucleotide sequence represented by SEQ ID NO: 2 in intestinal bacterial flora is decreased.

4. The agent according to any one of claims 1 to 3, wherein the agent is orally inoculated.
